# EUROPEAN PATENT APPLICATION

(11) **EP 4 484 549 A1**
(43) Date of publication of application: **01.01.2025**
(21) Application number: 23760014.3
(22) Date of filing: 22.02.2023
(51) Int. Cl.: C12N 1/20, C12N 1/21, C12N 15/01, C12N 15/31, C12Q 1/04, C12Q 1/6827, A23C 9/123, A23L 33/135

(54) **LACTIC ACID BACTERIA, LACTIC ACID BACTERIA COMPOSITION, PRODUCTION METHOD FOR LACTIC ACID BACTERIA, METHOD FOR IMPROVING ACID RESISTANCE OF LACTIC ACID BACTERIA, SCREENING METHOD FOR LACTIC ACID BACTERIA, AND PRODUCTION METHOD FOR FERMENTED MILK**

(30) Priority: 22.02.2022 JP 2022025816
(71) Applicant: MEIJI CO., LTD, Chuo-ku Tokyo 104-8306 (JP)
(72) Inventor: ISHIDA, Tatsuya, Hachioji-shi, Tokyo 192-0919 (JP); KOIZUMI, Akiko, Hachioji-shi, Tokyo 192-0919 (JP); YAMAMOTO, Eri, Hachioji-shi, Tokyo 192-0919 (JP)
(74) Representative: TBK
(86) International application number: PCT/JP2023/006381
(87) International publication number: WO 2023/163015

(57) **Abstract**

The present invention provides a method for improving acid tolerance of a lactic acid bacterium, including a step of artificially inhibiting a function of a kup gene of the lactic acid bacterium.

## Description

### [Technical Field]

The present invention relates to a lactic acid bacterium, a lactic acid bacterium composition, a method for producing a lactic acid bacterium, a method for improving acid tolerance of a lactic acid bacterium, a method for screening a lactic acid bacterium, and a method for producing fermented milk, and more specifically relates to providing a lactic acid bacterium and a lactic acid bacterium composition comprising the same, a method for producing a lactic acid bacterium, a method for improving acid tolerance of a lactic acid bacterium, a method for screening a lactic acid bacterium, and a method for producing fermented milk using these lactic acid bacteria and/or lactic acid bacterium compositions.

### [Background Art]

Lactic acid bacteria have long been used as microorganisms for producing fermented foods such as fermented milk like yogurt and pickles, but in recent years they have also attracted much attention as probiotics that provide various beneficial effects to the host, such as regulating the bacterial flora in the digestive tract to exert intestinal regulation effects, immune function improvement effects, and anti-tumor effects.

However, conventional lactic acid bacteria generally have low acid tolerance; for example, when orally ingested lactic acid bacteria pass through the gastric and bile acids, their survival rate in the intestines decreases due to the acids, resulting in the problem that the effects inherently provided by the lactic acid bacteria as probiotics cannot be sufficiently exhibited in the intestines. Therefore, several studies have been conducted so far with the aim of improving the acid tolerance of lactic acid bacteria. For example, Japanese Patent Application Publication No. 2005-160 (PTL 1) describes a method for improving the survivability of microorganisms including lactic acid bacteria in vivo in humans and animals by disrupting the hrcA gene of the microorganisms. Also, for example, Japanese Patent Application Publication No. 2001-204468 (PTL 2) describes acid-tolerant lactic acid-producing microorganisms comprising a recombinant vector incorporating therein a lactic acid dehydrogenase gene and promoter derived from a microorganism belonging to the genus Bifidobacterium or the genus Lactobacillus for the purpose of obtaining microorganisms with high lactic acid productivity even under acidic conditions.

### [Citation List]

### [Patent Literature]

[PTL 1] Japanese Patent Application Publication No. 2005-160
[PTL 2] Japanese Patent Application Publication No. 2001-204468

### [Summary of Invention]

### [Technical Problem]

However, factors involved in acid tolerance of lactic acid bacteria have not been reported sufficiently. Therefore, for example, conventional tests to determine whether lactic acid bacteria have excellent acid tolerance include artificial gastric juice tolerance tests (in vitro) and transit tolerance tests through the digestive tract of animals, but these methods also have the problem that it is difficult to test a large number of lactic acid bacterial strains at one time. Thus, further research on factors involved in acid tolerance of lactic acid bacteria has been desired.

The present invention has been made in view of the above problems in the prior art, and an object thereof is to provide a lactic acid bacterium that has sufficiently excellent tolerance to acids such as gastric acid and a lactic acid bacterium composition comprising the same, a method for producing a lactic acid bacterium with improved tolerance to acids, a method for improving acid tolerance of a lactic acid bacterium, a method for screening a lactic acid bacterium that has sufficiently excellent tolerance to acids, and a method for producing fermented milk using these lactic acid bacteria and/or lactic acid bacterium compositions.

### [Solution to Problem]

The present inventors have conducted intensive studies to achieve the above objective and succeeded in isolating lactic acid bacteria that show high survival rates in acid tolerance tests. Next, in order to explore factors involved in acid tolerance of lactic acid bacteria, genome analysis was conducted between lactic acid bacteria with high survival rates (highly acid-tolerant lactic acid bacteria) in the acid tolerance tests and conventional general lactic acid bacteria (i.e., lactic acid bacteria with low survival rates in acid tolerance tests: low acid-tolerant lactic acid bacteria). In this genome analysis, frameshift mutations and nonsense mutations in the kup gene that accompany amino acid mutations were extracted as mutations common to the highly acid-tolerant lactic acid bacteria. Furthermore, when the highly acid-tolerant lactic acid bacteria were subcultured multiple times to induce mutations, lactic acid bacteria that had lost acid tolerance were obtained; therefore, genome analysis was also conducted between these lactic acid bacteria and the highly acid-tolerant lactic acid bacteria. As a result, in the lactic acid bacteria that had lost acid tolerance, the frameshift mutations and nonsense mutations in the kup gene extracted in the highly acid-tolerant lactic acid bacteria were lost due to the induced mutations, and the nucleotide sequence was almost restored to that of the kup gene of the original low acid-tolerant lactic acid bacteria.

Therefore, from these findings, the present inventors have found that the kup gene is a factor involved in acid tolerance of lactic acid bacteria, and lactic acid bacteria in which the function of such kup gene is inhibited have sufficient tolerance to acids. Thus, it has also been found that by artificially inhibiting the function of the kup gene, improvement of the acid tolerance of lactic acid bacteria or inhibition of the function of the kup gene can be used as an indicator to easily screen lactic acid bacteria with sufficiently excellent tolerance to acids; this has led to the completion of the present invention.

Accordingly, the present invention provides the following aspects.
[1] A method for improving acid tolerance of a lactic acid bacterium, comprising a step of artificially inhibiting a function of a kup gene of the lactic acid bacterium.
[2] The method according to [1], wherein the lactic acid bacterium is a lactic acid bacterium of the family Lactobacillaceae.
[3] The method according to [1] or [2], wherein the lactic acid bacterium is a lactic acid bacterium of the genus Lactobacillus.
[4] A method for producing a lactic acid bacterium with improved acid tolerance, comprising a step of artificially inhibiting a function of a kup gene of a lactic acid bacterium.
[5] The production method according to [4], wherein the lactic acid bacterium is a lactic acid bacterium of the family Lactobacillaceae.
[6] The production method according to [4] or [5], wherein the lactic acid bacterium is a lactic acid bacterium of the genus Lactobacillus.
[7] A lactic acid bacterium in which a function of a gene is inhibited.
[8] The lactic acid bacterium according to [7], which is a lactic acid bacterium of the family Lactobacillaceae.
[9] The lactic acid bacterium according to [7] or [8], which is a lactic acid bacterium of the genus Lactobacillus.
[10] A lactic acid bacterium composition comprising the lactic acid bacterium according to any one of [7] to [9].
[11] The lactic acid bacterium composition according to [10], further comprising a lactic acid bacterium of the genus Streptococcus.
[12] The lactic acid bacterium composition according to [10] or [11], which is a lactic acid bacterium starter.
[13] The lactic acid bacterium composition according to [10] or [11], which is fermented milk.
[14] A method for screening a highly acid-tolerant lactic acid bacterium, comprising a selection step of selecting a lactic acid bacterium using inhibition of a function of a kup gene as an indicator.
[15] A method for producing fermented milk, comprising a fermentation step of adding any selected from the group consisting of the lactic acid bacterium obtained by the production method according to any one of [4] to [6], the lactic acid bacterium according to any one of [7] to [9], the lactic acid bacterium composition according to any one of [10] to [13], and the lactic acid bacterium selected by the screening method according to [14], to formula milk comprising raw material milk and performing fermentation to obtain fermented milk.
[16] The method for producing fermented milk according to [15], further comprising a step of adding a lactic acid bacterium of the genus Streptococcus to the formula milk.

### [Advantageous Effects of Invention]

According to the present invention, it becomes possible to provide a lactic acid bacterium that has sufficiently excellent tolerance to acids such as gastric acid and a lactic acid bacterium composition comprising the same, a method for producing a lactic acid bacterium with improved tolerance to acids, a method for improving acid tolerance of a lactic acid bacterium, a method for screening a lactic acid bacterium that has sufficiently excellent tolerance to acids, and a method for producing fermented milk using these lactic acid bacteria and/or lactic acid bacterium compositions.

### [Description of Embodiments]

Hereinafter, the present invention will be described in detail with reference to preferred embodiments thereof.

### <Lactic Acid Bacterium>

In the present invention, the term "lactic acid bacterium (or lactic acid bacteria)" refers to a collective term for microorganisms capable of assimilating glucose and producing lactic acid at a yield of 50% or more based on sugar, and as physiological characteristics, they are gram-positive cocci or rods, non-motile, basically lacking the ability to form spores (although some lactic acid bacteria, such as Bacillus coagulans, have exceptionally the ability to form spores), and possess features such as being catalase-negative.

Examples of the lactic acid bacterium according to the present invention include lactic acid bacteria of the family Streptococcaceae, lactic acid bacteria of the family Lactobacillaceae, lactic acid bacteria of the family Leuconostocaceae, and more specifically, lactic acid bacteria of the genus Lactobacillus, lactic acid bacteria of the genus Lacticaseibacillus, lactic acid bacteria of the genus Lactiplantibacillus, lactic acid bacteria of the genus Liquorilactobacillus, lactic acid bacteria of the genus Limosilactobacillus, lactic acid bacteria of the genus Levilactobacillus, lactic acid bacteria of the genus Lentilactobacillus, lactic acid bacteria of the genus Weissella, and other lactic acid bacilli; lactic acid bacteria of the genus Pediococcus, lactic acid bacteria of the genus Leuconostoc, lactic acid bacteria of the genus Lactococcus, lactic acid bacteria of the genus Streptococcus, lactic acid bacteria of the genus Enterococcus, and other lactic acid cocci; and lactic acid bacteria of the genus Bifidobacterium. Among these, lactic acid bacteria of the family Lactobacillaceae are preferable, and lactic acid bacteria of the genus Lactobacillus are more preferable.

Examples of lactic acid bacteria of the genus Lactobacillus include Lactobacillus delbrueckii, Lactobacillus nasalidis, Lactobacillus equicursoris, Lactobacillus helveticus, Lactobacillus acidophilus, Lactobacillus johnsonii, Lactobacillus gasseri, Lactobacillus paragasseri, Lactobacillus amylovorus, and Lactobacillus crispatus. In the present invention, each species includes subspecies thereof.

Among the lactic acid bacteria of the genus Lactobacillus, Lactobacillus delbrueckii is particularly preferable as the lactic acid bacterium of the present invention; examples of Lactobacillus delbrueckii include Lactobacillus delbrueckii subsp. bulgaricus (Bulgarian bacillus), Lactobacillus delbrueckii subsp. delbrueckii, Lactobacillus delbrueckii subsp. lactis, Lactobacillus delbrueckii subsp. indicus, Lactobacillus delbrueckii subsp. sunkii, and Lactobacillus delbrueckii subsp. jakobsenii.

It should be noted that according to the present invention, in addition to lactic acid bacteria, acid tolerance of bifidobacteria can also be improved and bifidobacteria with sufficiently excellent tolerance to acids can be easily screened by artificially inhibiting the function of the kup gene in bifidobacteria by using the inhibition of the function of the kup gene as an indicator. In the present invention, "bifidobacteria" refers to the general term for bacteria belonging to the genus Bifidobacterium in the order Bifidobacteriales, class Actinomycetes, which are Gram-positive, obligately anaerobic bacilli capable of producing acetic acid and lactic acid by metabolizing lactose and oligosaccharides.

### (kup Gene)

The lactic acid bacterium of the present invention is a lactic acid bacterium in which the function of the kup gene is inhibited. The "kup gene" is a gene widely conserved among microorganisms; in E. coli and others, it is known as a gene encoding a protein that functions as a potassium uptake transporter (Kup). However, the function of the protein (or amino acid sequence) encoded by the kup gene in lactic acid bacteria (particularly lactic acid bacteria of the genus Lactobacillus) has not been fully elucidated.

Examples of the kup gene according to the present invention include a gene encoding the amino acid sequence set forth in SEQ ID NO: 1, and a typical nucleotide sequence thereof is shown in SEQ ID NO: 2. The amino acid sequence set forth in SEQ ID NO: 1 and the nucleotide sequence set forth in SEQ ID NO: 2 are derived from Lactobacillus delbrueckii subsp. bulgaricus P2103001 (a bacterial strain deposited at Meiji Innovation Center of Meiji Co., Ltd. (postal code 192-0919, 1-29-1 Nanakuni, Hachioji City, Tokyo, Japan)).

The amino acid sequence encoded by the kup gene is conserved with high identity among lactic acid bacteria (particularly lactic acid bacteria of the family Lactobacillaceae and preferably lactic acid bacteria of the genus Lactobacillus); examples of the kup gene according to the present invention also include genes encoding amino acid sequences shown in Table 1 below. It should be understood that nucleotide sequences of the genes encoding these amino acid sequences may have individual variations at the strain level due to polymorphisms and others. Table 1 shows the accession numbers of typical amino acid sequences encoded by the kup genes of lactic acid bacteria, the amino acid identity (%) to the amino acid sequence set forth in SEQ ID NO: 1, and the names of the lactic acid bacteria from which the amino acid sequences originate.

**[Table 1]**

| Lactic Acid Bacteria | Identity (%) | Accession No. |
|---|---|---|
| Lactobacillus sp. HMSC08B12 | 94.63 | WP_070488699 |
| Lactobacillus porci | 93.62 | WP_154549030 |
| Lactobacillus nasalidis | 95.55 | WP_201332156 |
| Lactobacillus nasalidis | 95.34 | WP_201336306 |
| Lactobacillus nasalidis | 93.85 | WP_201336177 |
| Lactobacillus equicursoris | 92.14 | WP_154486640 |
| Lactobacillus equicursoris | 92.14 | WP_008463572 |
| Lactobacillus equicursoris | 91.99 | WP_009557630 |
| Lactobacillus delbrueckii subsp. lactis | 97.34 | CDR76610 |
| Lactobacillus delbrueckii subsp. lactis | 97.19 | CDR82863 |
| Lactobacillus delbrueckii subsp. lactis | 94.11 | CDR79854 |
| Lactobacillus delbrueckii subsp. lactis | 93.59 | AZA16843 |
| Lactobacillus delbrueckii subsp. jakobseniiZN7a-9 = DSM 26046 | 94.11 | KRO19959 |
| Lactobacillus delbrueckii subsp. indicus DSM 15996 | 93.83 | KRL77314 |
| Lactobacillus delbrueckii subsp. delbrueckii DSM 20074= JCM 1012 | 93.81 | KRK25837 |
| Lactobacillus delbrueckii subsp. bulgaricus | 99.56 | MBT8925462 |
| Lactobacillus delbrueckii subsp. bulgaricus | 98.96 | MBT9058086 |
| Lactobacillus delbrueckii subsp. bulgaricus | 98.52 | MBT8883881 |
| Lactobacillus delbrueckii subsp. bulgaricus | 98.22 | MBT8855447 |
| Lactobacillus delbrueckii subsp. bulgaricus | 98.22 | MBT8815790 |
| Lactobacillus delbrueckii subsp. bulgaricus | 97.78 | MBT8849340 |
| Lactobacillus delbrueckii | 100 | WP_014564539 |
| Lactobacillus delbrueckii | 99.41 | WP_003621259 |
| Lactobacillus delbrueckii | 99.41 | WP_035176007 |
| Lactobacillus delbrueckii | 99.26 | WP_041806388 |
| Lactobacillus delbrueckii | 99.11 | WP_077300791 |
| Lactobacillus delbrueckii | 99.11 | WP_114893777 |
| Lactobacillus delbrueckii | 99.11 | WP_119906298 |
| Lactobacillus delbrueckii | 98.82 | TXJ89545.1 |
| Lactobacillus delbrueckii | 98.52 | WP_156301229 |
| Lactobacillus delbrueckii | 98.52 | WP_216698217 |
| Lactobacillus delbrueckii | 97.34 | WP_130183223 |
| Lactobacillus delbrueckii | 97.19 | WP_072538274 |
| Lactobacillus delbrueckii | 94.78 | WP_050952002 |
| Lactobacillus delbrueckii | 94.63 | WP_086356073 |
| Lactobacillus delbrueckii | 94.49 | WP_003614473 |
| Lactobacillus delbrueckii | 94.19 | WP_072546956 |
| Lactobacillus delbrueckii | 94.04 | WP_141373048 |
| Lactobacillus delbrueckii | 93.74 | WP_129333056 |
| Lactobacillus delbrueckii | 93.59 | WP_035183966 |
| Lactobacillus delbrueckii | 93.44 | WP_072544255 |

### (Function Inhibition of kup Gene)

In the present invention, "the function of the kup gene is inhibited (function inhibition of kup gene)" means that a mutation accompanied by a function-inhibiting amino acid mutation has occurred in the kup gene and/or the expression of the kup gene is decreased. Although the specific function of the amino acid sequence encoded by the kup gene (particularly that of lactic acid bacteria of the genus Lactobacillus) has not been fully elucidated, the present inventors have found that acid tolerance can be improved by inhibiting the function of the kup gene in lactic acid bacteria, i.e., at least the function to encode and express the correct amino acid sequence (e.g., the amino acid sequence set forth in SEQ ID NO: 1 or Table 1). Therefore, it can be said that the "function of the kup gene" in the present invention includes at least an acid-sensitive function. In the present invention, the term "inhibition" of function also includes deletion of the function.

### [Mutation Accompanied by Function-Inhibiting Amino Acid Mutation]

In the present invention, examples of the "function-inhibiting amino acid mutation" include, but are not particularly limited to, substitution, deletion, insertion, and/or addition of one or more amino acids, as long as the acid-sensitive function is inhibited (including deleted), that is, the acid tolerance of the lactic acid bacterium is improved; more specific examples include substitution, deletion, insertion, and/or addition of one or more amino acids in the amino acid sequence set forth in SEQ ID NO: 1 or Table 1, or amino acid sequences corresponding thereto, and preferably substitution, deletion, insertion, and/or addition of one or more amino acids in the amino acid sequence set forth in SEQ ID NO: 1.

Note that in the present invention, an amino acid sequence or region "corresponding" to an amino acid sequence or region refers to an amino acid sequence or region that is aligned with a control amino acid sequence or region (e.g., the amino acid sequence set forth in SEQ ID NO: 1, the amino acid sequences set forth in Table 1, or the amino acid sequence set forth in SEQ ID NO: 3 mentioned below) when amino acid sequences are aligned using amino acid sequence analysis software (GENETYX-MAC, Sequencher, etc.) or BLAST (Basic Local Alignment Search Tool at the National Center for Biological Information) or the like (e.g., parameters: default (that is, initially set values)).

Examples of such mutations of the kup gene accompanied by function-inhibiting amino acid mutations include missense mutations, nonsense mutations, frameshift mutations, and combinations thereof due to substitution, deletion, insertion, and/or addition of one or more bases in the nucleotide sequence of the kup gene, and also include substitution and/or deletion of multiple or all bases by gene knockout or the like.

Preferably, function-inhibiting amino acid mutations by which the acid-sensitive function can be more sufficiently inhibited, that is, the acid tolerance of lactic acid bacteria can be more sufficiently improved, are those with substituted, deleted, inserted, and/or added amino acids, preferably substituted and/or deleted amino acids, of 46 residues or more, more preferably 60 residues or more, and further preferably 70 residues or more. Such function-inhibiting amino acid mutations also further preferably include substitution, deletion, insertion, and/or addition in all or part of at least the C-terminal domain of the amino acid sequence encoded by the kup gene, and particularly preferably include deletion of all or part of at least the C-terminal domain.

In the present invention, the C-terminal domain of the amino acid sequence encoded by the kup gene refers to the region at the C-terminal side at and after the 450th Gly in the amino acid sequence set forth in SEQ ID NO: 1 and regions corresponding thereto. SEQ ID NO: 3 shows a typical sequence of the C-terminal domain (at and after the 450th Gly in the amino acid sequence set forth in SEQ ID NO: 1), and SEQ ID NO: 4 shows a typical nucleotide sequence encoding it.

Examples of deletion of all or part of the C-terminal domain include, more specifically, deletion of preferably 20% or more, and more preferably 26% or more, 30% or more, 50% or more, 70% or more, 80% or more, or 100% of the amino acids in the C-terminal domain (227 amino acids in SEQ ID NO: 3).

Examples of such function-inhibiting amino acid mutations include, but are not limited to, substitution and/or deletion of 46 or more residues (preferably 60 or more residues or 70 or more residues) of amino acids in the amino acid sequence set forth in SEQ ID NO: 1 or Table 1, or amino acid sequences corresponding thereto (preferably the amino acid sequence set forth in SEQ ID NO: 1); and deletion of 20% or more (preferably 26% or more, 30% or more, 50% or more, 70% or more, 80% or more, or 100%) of amino acids in the amino acid sequence set forth in SEQ ID NO: 3 or an amino acid sequence corresponding thereto (preferably the amino acid sequence set forth in SEQ ID NO: 3).

Methods for detecting "mutations accompanied by function-inhibiting amino acid mutations" in the kup gene of lactic acid bacteria in the present invention are not particularly limited and conventionally known methods can be appropriately applied; examples include the following methods. Note that "mutation detection" in a gene in the present invention principally signifies detecting mutations on the genomic DNA. However, since the mutations on this genomic DNA are reflected in the changes of bases in transcription products and changes of amino acids in translation products, it also encompasses detecting these changes in these transcription and translation products (that is, indirect detection at the transcription and translation levels).

An embodiment of the detection method is a method of directly determining the nucleotide sequence in the gene region of the kup gene and detecting mutations by comparing it with a control. In the present invention, the "gene region of the kup gene" means a certain region on genomic DNA comprising at least the kup gene, which may also comprise non-translated regions as expression control regions (e.g., promoter region, enhancer region) of the kup gene and 5'-terminal non-translated regions of the kup gene, in addition to translated regions.

In the present embodiment, first, DNA samples are prepared from the target lactic acid bacteria. Examples of DNA samples include genomic DNA samples, and cDNA samples prepared by reverse transcription from RNA. There are no particular limitations on the method for extracting genomic DNA or RNA from the lactic acid bacteria and the method for preparing cDNA, and for each of them, any known method or a method similar thereto can be appropriately employed. Then, DNA including the gene region of the kup gene is isolated, and the nucleotide sequence of the isolated DNA is determined. The isolation of this DNA can be performed, for example, using a pair of oligonucleotide primers designed to encompass all or part (preferably all) of the gene regions of the kup gene by PCR or the like with genomic DNA or RNA as a template. Those skilled in the art can design suitable oligonucleotide primers by a conventional method based on nucleotide sequences of the kup gene (preferably the nucleotide sequence set forth in SEQ ID NO: 2 or nucleotide sequences encoding the amino acid sequences set forth in Table 1) available from public databases (Genbank, etc.). The determination of the nucleotide sequence of the isolated DNA can be performed by a method known to those skilled in the art, such as the Maxam-Gilbert method or the Sanger method. DNA libraries may also be created from the prepared genomic DNA or cDNA, and the nucleotide sequence of the entire genomic DNA may be determined using a next-generation sequencer or the like.

When a mutation in the gene region of the kup gene is detected in the target lactic acid bacterium by comparing the determined DNA or cDNA nucleotide sequence with a control, it can be determined that the function of the kup gene is inhibited depending on the type and/or degree of the mutation. As the type and/or degree of the mutation in the gene region of the kup gene detected at this time that enables more sufficient improvement of the acid tolerance of the lactic acid bacterium, it is preferable that the mutation is of the type and/or degree accompanied by the amino acid mutation mentioned as a preferable embodiment of the function-inhibiting amino acid mutation. Examples of the control here include known nucleotide sequences of the kup gene, and more specifically, for example, the nucleotide sequence set forth in SEQ ID NO: 2 and nucleotide sequences encoding the amino acid sequences set forth in Table 1. Also, in the case of the production method or acid tolerance improving method of the present invention described below, examples of the control include the nucleotide sequence of the kup gene of the lactic acid bacterium before artificially inhibiting the function of the kup gene.

Examples of other embodiments of the aforementioned detection method include the following methods:
a method utilizing restriction fragment length polymorphism (RFLP) where DNA comprising all or part (preferably all) of the gene regions of the kup gene is amplified, and the size of the DNA fragments of the amplification products by restriction enzymes is compared with a control, or PCR-RFLP method;
the PCR-SSCP (single-strand conformation polymorphism) method where the amplification products are dissociated into single-stranded DNA, separated on a non-denaturing gel, and compared in mobility on the gel with the control;
the denaturant gradient gel electrophoresis (DGGE) method where the amplification products are separated on a gel with a concentration gradient of DNA denaturant, and compared in mobility on the gel with the control; the MALDI-TOF/MS method where an "oligonucleotide primer having bases on the 1-base 3'-side among all or part (preferably all) of the bases of the gene regions of the kup gene and a nucleotide sequence complementary to the nucleotide sequence on the 3'-side thereof" is used with the above DNA sample as a template to perform ddNTP primer extension reaction, and the genotype determined by mass measurement is compared with a control;
the pyrosequencing method where the amplification products are dissociated into single strands and only one of the strands is separated, and extension reaction is performed base by base from the vicinity of all or part (preferably all) of the bases of the gene regions of the kup gene, and the pyrophosphate produced at that time is measured and compared with the control; and the AcycloPrime method where in the presence of fluorescent-labeled nucleotides, a one-base extension reaction is performed with an "oligonucleotide primer having bases on the 1-base 3'-side among all or part (preferably all) of the bases of the gene regions of the kup gene and a nucleotide sequence complementary to the nucleotide sequence on the 3'-side thereof" using the above amplification products as a template, and the fluorescence anisotropy is measured and compared with the control, and the SNuPE method where the type of base used in the one-base extension reaction is determined and compared with the control.

In each detection method, if the detection result is different from the control after comparing the obtained result with the control, for example, if a result indicating that the chain length of the kup gene is shorter than the control is obtained, it can be determined that the gene region of the kup gene is mutated in the lactic acid bacterium, that is, the function of the kup gene is inhibited. As the chain length of the kup gene that enables more sufficient improvement of the acid tolerance of the lactic acid bacterium, it is preferable that the chain length is accompanied by the amino acid mutation mentioned as a preferable embodiment of the function-inhibiting amino acid mutation. Examples of the control here include DNA samples of the kup gene known to have no mutations, and more specifically, for example, standard DNA samples artificially synthesized based on nucleotide sequences such as the nucleotide sequence set forth in SEQ ID NO: 2 and nucleotide sequences encoding the amino acid sequences set forth in Table 1. Also, in the case of the production method or acid tolerance improving method of the present invention described below, examples of the control include DNA samples extracted from lactic acid bacteria before artificially inhibiting the function of the kup gene.

### [Decreased Expression of kup Gene]

In the present invention, "the expression of the kup gene is decreased (decreased expression of the kup gene)" means that substantially the full length of the amino acid sequence encoded by the kup gene is not expressed. As "substantially the full length of the amino acid sequence encoded by the kup gene" herein, it is preferable that the length is 70% or more, 80% or more, 90% or more, 93% or more, 95% or more, or 100% of the amino acid sequence set forth in SEQ ID NO: 1 or Table 1, or amino acid sequences corresponding thereto (preferably the amino acid sequence set forth in SEQ ID NO: 1).

The fact that substantially the full length of the amino acid sequence encoded by the kup gene is not expressed can be detected by, for example, Western blotting, SDS-PAGE, and amino acid sequencing.

Furthermore, it is known in the art that decreased expression of genes is due to factors such as excessive methylation of the promoter. Therefore, another embodiment of the detection method may be, for example, a method of detecting expression at the transcription level of the kup gene by using methylation of the kup gene promoter as an indicator. For the detection of promoter methylation, for example, known methods can be utilized such as a method that directly detects changes in the nucleotide sequence after bisulfite treatment which has the activity to convert methylated cytosine to uracil by nucleotide sequence determination, and a method that indirectly detects using a restriction endonuclease that can recognize (cut) the nucleotide sequence before bisulfite treatment but cannot recognize (cut) the nucleotide sequence after bisulfite treatment. Depending on the degree of methylation, it can be determined that expression is not detected at the transcription level, that is, the function of the kup gene is inhibited.

In the present invention, as a method for detecting the "function inhibition of the kup gene" of the lactic acid bacteria, any one of the above detection methods can be employed, and two or more thereof may be combined. Among these, from the viewpoint of accuracy and convenience, it is preferable to detect function inhibition of kup gene by detecting a mutation accompanied by a function-inhibiting amino acid mutation in the kup gene in the target lactic acid bacterium, and it is more preferable to detect the mutation by directly determining the nucleotide sequence of the kup gene and comparing it with a control.

The lactic acid bacterium of the present invention may be a single species or a combination of two or more species. More specifically, the lactic acid bacterium of the present invention includes Lactobacillus delbrueckii (L. delbrueckii) in which the kup gene is functionally inhibited, and examples include Lactobacillus delbrueckii species such as L. delbrueckii subsp. bulgaricus OLL205405 (hereinafter, optionally referred to as "strain f") identified by accession number NITE BP-03574 and L. delbrueckii subsp. bulgaricus OLL205406 (hereinafter, optionally referred to as "strain i") identified by accession number NITE BP-03575.

Strain f and strain i have a mutation accompanied by a function-inhibiting amino acid mutation as function inhibition of kup gene. Specifically, strain f has a mutation of substituting the 1850th G to A in the nucleotide sequence set forth in SEQ ID NO: 2, which involves a nonsense mutation where the codon (TGG) corresponding to the 617th Trp in the amino acid sequence set forth in SEQ ID NO: 1 becomes a termination codon (TAG). Strain i has a mutation of deleting the 521st T in the nucleotide sequence set forth in SEQ ID NO: 2, which involves a frameshift mutation and nonsense mutation where the amino acids at and after the 174th Phe in the amino acid sequence set forth in SEQ ID NO: 1 are substituted, and further, the codon corresponding to the 190th Leu in the amino acid sequence set forth in SEQ ID NO: 1 becomes a termination codon.

Strain f is deposited with (1) identification label: L. delbrueckii subsp. bulgaricus OLL205405 and (2) accession number: NITE BP-03574 on (3) Deposit date: December 16, 2021, and strain i is deposited with (1) identification label: L. delbrueckii subsp. bulgaricus OLL205406 and (2) accession number: NITE BP-03575 on (3) Deposit date: December 16, 2021, with both strains deposited at (4) Depositary institution: National Institute of Technology and Evaluation NITE Patent Microorganisms Depositary (postal code 292-0818, 2-5-8, Kazusa-kamatari, Kisarazu City, Chiba Prefecture, Room 122). The Lactobacillus delbrueckii identified by these accession numbers may be a subcultured strain of the same strain, an artificial mutant strain, a natural mutant strain, a genetically modified strain, a derivative strain, or the like of the same strain or a subcultured strain thereof, as long as the effects of the present invention are not impaired, and may have mutations other than the mutation of the kup gene with the function-inhibiting amino acid mutation.

### (Acid Tolerance)

The lactic acid bacterium of the present invention is a lactic acid bacterium with excellent acid tolerance (hereinafter optionally referred to as "highly acid-tolerant lactic acid bacterium" in the present specification) because the kup gene is functionally inhibited. In the present invention, a "highly acid-tolerant lactic acid bacterium" indicates a lactic acid bacterium capable of surviving even at low pH (e.g., pH 3.5 or less, preferably pH 3.0 or less).

The acid tolerance of a lactic acid bacterium can actually be confirmed and evaluated by, for example, the following acid tolerance test. That is, first, the target lactic acid bacterium is activation-cultured in MRS medium at 37°C under anaerobic conditions for 18 hours as necessary, and then cultured in MRS medium at 37°C under anaerobic conditions for 18 hours. Next, the bacterial bodies recovered after culturing are added to artificial gastric juice (pH 3.0, 0.2% NaCl, 0.175% pepsin) and treated with acid at 37°C for 2 hours; then, the survival rate ((viable bacterial count after acid treatment/viable bacterial count before acid treatment) × 100(%)) is calculated, and it can be confirmed and evaluated that the higher the survival rate, the higher the acid tolerance. In the present invention, the "highly acid-tolerant lactic acid bacterium" only needs to be viable at the low pH, and may have a moderate survival rate. More specifically, in the acid treatment test, the highly acid-tolerant lactic acid bacterium preferably has a survival rate of 20% or more, more preferably 30% or more, and further preferably 50% or more.

### <Method for Producing Lactic Acid Bacterium and Method for Improving Acid Tolerance of Lactic Acid Bacterium>

Since a lactic acid bacterium with a functionally inhibited kup gene has excellent acid tolerance, it is possible to improve the acid tolerance of the lactic acid bacterium by artificially inhibiting the function of the kup gene of the lactic acid bacterium. Therefore, the present invention also provides a method for producing a lactic acid bacterium with improved acid tolerance, including a step of artificially inhibiting the function of the kup gene of a lactic acid bacterium, and a method for improving acid tolerance of a lactic acid bacterium, including a step of artificially inhibiting the function of the kup gene of a lactic acid bacterium.

The kup gene as a target in the production method or acid tolerance improvement method of the present invention is typically a gene encoding the amino acid sequence set forth in SEQ ID NO: 1 or Table 1 as described above; however, since the nucleotide sequence of the kup gene may be changed by natural gene mutations, hybridization, and the like, the kup gene as a target in the production method or acid tolerance improvement method of the present invention also includes a gene of a lactic acid bacterium that is orthologous to the gene encoding the amino acid sequence set forth in SEQ ID NO: 1.

In the present invention, "ortholog" encompasses not only genes having the same function among different species, but also genes having the same function among different strains even in the same species. More specifically, in the present invention, a "gene orthologous to a gene encoding the amino acid sequence set forth in SEQ ID NO: 1" indicates "a gene encoding the amino acid sequence having the highest identity to the amino acid sequence set forth in SEQ ID NO: 1" in a certain lactic acid bacterium, which corresponds to "a gene encoding an amino acid sequence corresponding to the amino acid sequence set forth in SEQ ID NO: 1" in the present specification. The sequence of such a gene can be obtained, for example, by acquiring the nucleotide sequence of the total genomic DNA of the target lactic acid bacterium, and searching using the above amino acid sequence analysis software, BLAST, or the like with the amino acid sequence of SEQ ID NO: 1 as a query. In addition to determining and acquiring the nucleotide sequence of the total genomic DNA of the target lactic acid bacterium by an appropriately known method (e.g., whole genome sequencing), it can also be acquired from a database such as GenBank (NCBI).

The lactic acid bacterium to be targeted in the production method or acid tolerance improvement method of the present invention is preferably a lactic acid bacterium whose kup gene function is not inhibited or possibly inhibited, but such a lactic acid bacterium whose kup gene function is inhibited or possibly inhibited can be selected by, for example, the above acid tolerance test or the method for screening a lactic acid bacterium of the present invention described below.

As a method for artificially inhibiting the function of the kup gene in a lactic acid bacterium, examples include a method for introducing a mutation accompanied by a function-inhibiting amino acid mutation into the kup gene, and/or a method for decreasing the expression of the kup gene. The mutation accompanied by a function-inhibiting amino acid mutation and the decrease in expression are as described above including preferable embodiments thereof; as a method for artificially inhibiting the function of the kup gene according to the present invention, conventionally known methods or methods analogous thereto can be appropriately employed according to the purpose and degree.

Examples of methods for artificially introducing a mutation accompanied by a function-inhibiting amino acid mutation into the kup gene include gene knockout methods; gene targeting methods utilizing homologous recombination, site-directed mutagenesis methods such as the Kunkel method and SOE-PCR; genome editing methods (methods using site-directed nucleases (meganucleases; zinc finger nucleases; TALEN; PPR; CRISPR-Cas such as CRISPR-Cas9, etc.)); methods for inducing mutations by repeated subcultures; methods for inducing mutations by irradiation with UV, X-rays, gamma rays, etc.; methods for inducing mutations by treatment with chemical agents (e.g., nitrosoguanidine, diethyl sulfate, and nitrite); methods for inducing mutations by exposure to gastric acid or artificial digestive fluids; and combinations of two or more thereof.

Examples of methods for decreasing the expression of the kup gene include knockdown methods, promoter methylation, expression inhibition by antisense RNA expression (van der Krol et al., Nature 333: 866, 1988), expression inhibition by RNA interference (RNAi) using double-stranded RNA having a sequence corresponding to the kup gene, and combinations of two or more thereof.

In the production method or acid tolerance improving method of the present invention, the acid tolerance only needs to be improved compared to the lactic acid bacterium before the step of artificially inhibiting the function of the kup gene; the improvement in acid tolerance can be confirmed by, for example, the survival rate calculated in the above acid tolerance test being higher than that of the lactic acid bacterium before the step.

The production method or acid tolerance improving method of the present invention may further include, for example, a step of confirming that the lactic acid bacterium having been subjected to the step of artificially inhibiting the function of the kup gene is indeed the highly acid-tolerant lactic acid bacterium by carrying out the above acid tolerance test.

### <Method for Screening Lactic Acid Bacterium>

Since lactic acid bacteria with inhibited kup gene function have excellent acid tolerance, the present invention also provides a method for screening a highly acid-tolerant lactic acid bacterium, including a selection step of selecting a lactic acid bacterium using inhibition of the function of the kup gene as an indicator. The screening method of the present invention includes not only a method for screening lactic acid bacteria that are actually highly acid-tolerant but also a method for screening lactic acid bacteria that are likely to be highly acid-tolerant; it may also be a method for screening lactic acid bacteria at a level where moderate acid tolerance can be expected.

In the selection step, for example, whether or not the function of the kup gene is inhibited (i.e., whether or not a mutation accompanied by a function-inhibiting amino acid mutation is detected in the kup gene and/or decreased expression of the kup gene is detected) is determined by at least one of the detection methods for function inhibition of kup gene mentioned above, and lactic acid bacteria in which function inhibition of kup gene is detected are selected as the highly acid-tolerant lactic acid bacteria (including lactic acid bacteria with moderate acid tolerance) or lactic acid bacteria that are likely to be highly acid-tolerant. The determination method is as described above for each detection method, including preferable embodiments thereof.

The screening method of the present invention may further include, for example, a step of confirming that the screened lactic acid bacterium is indeed the highly acid-tolerant lactic acid bacterium by carrying out the above acid tolerance test.

### <Lactic Acid Bacterium Composition>

The term "lactic acid bacterium composition (or lactic acid bacteria composition)" refers to a composition comprising lactic acid bacteria, without particular limitations, and as the lactic acid bacteria composition of the present invention, it suffices to comprise at least the lactic acid bacteria of the present invention, and may be composed solely of the lactic acid bacteria of the present invention (including a combination of two or more types of lactic acid bacteria of the present invention). The present invention also provides a lactic acid bacterium composition comprising the lactic acid bacterium produced by the method for producing a lactic acid bacterium of the present invention or the lactic acid bacterium selected by the method for screening lactic acid bacterium. The lactic acid bacterium composition of the present invention encompasses, for example, the following lactic acid bacterium starter and fermented milk.

As the lactic acid bacterium composition of the present invention, it is also acceptable to further comprise additional components other than the lactic acid bacteria of the present invention, and these additional components are not particularly limited and may include solvents such as water; cultures such as the supernatant of the culture fluid after the lactic acid bacteria have finished culturing, and culture medium components; concentrated, diluted, dried, frozen forms of the culture, and the like, and these can be one or in combinations of two or more.

### (Lactic Acid Bacterium Starter)

The lactic acid bacterium starter (or lactic acid bacteria starter) of the present invention, which comprises at least the lactic acid bacteria of the present invention, can be suitably used for the method for producing fermented milk of the present invention described later. As such lactic acid bacterium starter of the present invention, it may be composed solely of the lactic acid bacteria of the present invention, or it may further comprise the aforementioned additional components, and additional lactic acid bacteria, yeast, bifidobacteria, etc.

Regarding additional lactic acid bacteria, yeast, and bifidobacteria, known lactic acid bacteria, yeast, and bifidobacteria that have conventionally been included in fermented milk are mentioned. Examples of additional lactic acid bacteria include lactic acid bacteria other than the lactic acid bacteria of the present invention, and these can be one or in combinations of two or more. Among these, if the lactic acid bacteria of the present invention are lactic acid bacteria of the genus Lactobacillus, as a lactic acid bacteria starter of the present invention, it is preferable to further comprise lactic acid bacteria of the genus Streptococcus other than the lactic acid bacteria of the present invention. In addition, the lactic acid bacterium starter of the present invention also preferably comprises lactic acid bacteria of the genus Lactobacillus, which is the lactic acid bacterium of the present invention, and lactic acid bacteria of the genus Streptococcus, which is the lactic acid bacterium of the present invention.

Lactic acid bacteria of the genus Streptococcus are aerobically Gram-positive cocci, and examples thereof include Streptococcus thermophilus and Streptococcus salivarius. Among these lactic acid bacteria of the genus Streptococcus, those belonging to Streptococcus thermophilus are preferable.

The lactic acid bacterium starter of the present invention may be in a liquid state or in a solid state such as a frozen state or a dry powder, and may further comprise additional components. Examples of the further additional components that can be comprised in the lactic acid bacterium starter include fermentation promoting substances (such as formic acid and nucleic acids); protective agents (such as saccharides); and medium components (such as milk and whey), and may be one of these or a combination of two or more thereof.

The content of the lactic acid bacteria of the present invention in the lactic acid bacteria starter of the present invention is not particularly limited, but preferably 0.01 to 100% by mass, and more preferably 0.1 to 90% by mass. Also, in terms of the viable bacterial count, it is preferable to be 1 × 10⁷ cfu/g or more, and more preferably 1 × 10⁷ to 1 × 10¹¹ cfu/g. Furthermore, in the case of further comprising additional lactic acid bacteria (preferably lactic acid bacteria of the genus Streptococcus other than the lactic acid bacterium of the present invention), the ratio of the content of the lactic acid bacteria of the present invention in the lactic acid bacteria starter and the content of the additional lactic acid bacteria is preferably 1:0.1 to 1:100, more preferably 1:1 to 1:10, in a bacterial count ratio (in terms of viable bacterial count, hereinafter the same). Furthermore, when the lactic acid bacterium starter of the present invention is a combination of a lactic acid bacterium of the genus Lactobacillus as the lactic acid bacterium of the present invention and a lactic acid bacterium of the genus Streptococcus as the lactic acid bacterium of the present invention, the ratio of the content of the lactic acid bacteria of the genus Lactobacillus to the content of the lactic acid bacteria of the genus Streptococcus in the lactic acid bacterium starter is preferably 1:0.1 to 1:100, more preferably 1:1 to 1:10, in a bacterial count ratio (in terms of viable bacterial count, hereinafter the same).

The lactic acid bacteria starter of the present invention may, for example, be combined with a composition comprising the additional lactic acid bacteria (second lactic acid bacteria composition) to form a lactic acid bacterium starter kit including the lactic acid bacterium starter of the present invention and the second lactic acid bacteria composition. Additionally, it may be, for example, in the form of a lactic acid bacterium starter kit including additives for producing fermented milk (such as fermentation promoting substances and protective agents described above), containers, and usage instructions for the lactic acid bacterium starter.

### (Fermented Milk)

The fermented milk of the present invention comprises at least the lactic acid bacterium of the present invention, and for example, can be obtained by the method for producing fermented milk of the present invention described later. The present invention also provides fermented milk comprising the lactic acid bacterium produced by the method for producing a lactic acid bacterium of the present invention or the lactic acid bacterium selected by the method for screening lactic acid bacterium. Since the lactic acid bacterium comprised in the fermented milk of the present invention has excellent acid tolerance, for example, even when the fermented milk is orally ingested and exposed to acids such as gastric acid, it can reach the intestines with a high survival rate.

The fermented milk of the present invention is not particularly limited, and examples thereof include fermented milk that satisfies the standards for "fermented milk" according to the Ministerial Ordinance on Milk and Milk Products (more specifically, the content of milk solids-not-fat is 8.0% or more, and the lactic acid bacteria count or yeast count (preferably the lactic acid bacteria count (more preferably the count of the lactic acid bacteria of the present invention), hereinafter the same) is 10 million/mL or more). In addition, the fermented milk of the present invention also includes those that satisfy the standards for "milk products and fermented milk drinks" (more specifically, the content of milk solids-not-fat is 3.0% or more, and the lactic acid bacteria count or yeast count is 10 million/mL or more); those that satisfy the standards for "fermented milk drinks" (more specifically, the content of milk solids-not-fat is 3.0% or more, and the lactic acid bacteria count or yeast count is 1 million/mL or more), according to the above Ministerial Ordinance on Milk and Milk Products. Note that the milk solids-not-fat refers to the remaining components (mainly such as proteins, lactose, and minerals) obtained by subtracting the fat content from the total milk solids, and the lactic acid bacteria and yeast count are viable bacterial counts measured using the test method stipulated in the Ministerial Ordinance on Milk and Milk Products and the method using the BCP-added plate count agar medium, and in the case of fermented milk that has undergone partial sterilization, they are in terms of the viable bacterial counts measured by the aforementioned test method before the partial sterilization.

The fermented milk of the present invention may be a fermented product after the fermentation step of the method for producing fermented milk described later or may be obtained by partially sterilizing the fermented product (such as pulverization or heat treatment), or may be obtained by, for example, concentrating, diluting, drying, or freezing them. Note that in the present invention, when the fermented milk is partially sterilized, the lactic acid bacteria count in the fermented milk is in terms of viable bacterial count. The lactic acid bacteria comprised in the fermented milk of the present invention include not only viable bacteria but also dead bacteria, as well as disrupted products and heat-treated products of lactic acid bacteria, and concentrates, dilutions, dried products, and frozen products thereof. The fermented milk of the present invention preferably comprises at least viable bacteria of lactic acid bacteria, and more preferably comprises the lactic acid bacteria of the present invention as viable bacteria.

The fermented milk of the present invention comprises components derived from the lactic acid bacteria of the present invention and the raw material milk (preferably formula milk) described later. Moreover, within the scope not inhibiting the effects of the present invention, it may further comprise additional components listed in the lactic acid bacterium starter mentioned above, as well as additional lactic acid bacteria, yeast, bifidobacteria, and the like. In addition, it may comprise various components that can be comprised in food and drink. Further components that can be comprised in fermented milk are not particularly limited, and examples thereof include saccharides, sugar alcohols, minerals, vitamins, proteins, peptides, amino acids, organic acids, pH adjusters, starches and modified starches, dietary fibers, fruits and vegetables and processed products thereof, animal and plant crude drug extracts, naturally-derived polymers (such as collagen, hyaluronic acid, and chondroitin), oils and/or fats, thickeners, emulsifiers, solvents, surfactants, gelling agents, stabilizers, buffers, suspending agents, thickening agents, excipients, disintegrators, binders, flow agents, preservatives, colorants, fragrances, corrigents, and sweeteners, and may be one of these or a combination of two or more thereof.

Such fermented milk is preferably yoghurt, cheese, fermented cream, fermented butter, and the like, and particularly preferably yogurt. Specific examples of the yogurt include set type yogurt (solid fermented milk) such as plain yogurt, soft-type yogurt (pasty fermented milk), and drink type yogurt (liquid fermented milk), and frozen yogurt using these ingredients may also be used. In addition, the fermented milk of the present invention can also be used as an ingredient for fermented food such as cheese, fermented cream, fermented butter, and kefir. Furthermore, the fermented milk of the present invention can also be used as a lactic acid bacteria starter in the method for producing fermented milk below.

### <Method for Producing Fermented Milk>

The method for producing fermented milk of the present invention includes a fermentation step of adding at least one of the lactic acid bacterium of the present invention, the lactic acid bacterium produced by the method for producing a lactic acid bacterium of the present invention, the lactic acid bacterium selected by the method for screening a lactic acid bacterium of the present invention, and the lactic acid bacterium composition of the present invention, to formula milk comprising raw material milk and performing fermentation to obtain fermented milk. In the case of using the lactic acid bacterium produced by the method for producing a lactic acid bacterium of the present invention or selected by the method for screening a lactic acid bacterium, the method for producing fermented milk of the present invention may further include the step of artificially inhibiting the function of the kup gene or the selection step, but these steps only need to be performed once initially.

### (Formula Milk)

The formula milk according to the present invention comprises raw material milk. The raw material milk preferably comprises lactose, and examples thereof include raw milk (such as milk of cow, water buffalo, sheep, goat, and the like), partially sterilized milk, whole milk, skim milk, whey, and processed products thereof (such as whole milk powder, full-fat concentrated milk, skimmed milk powder, skimmed concentrated milk, condensed milk, whey powder, buttermilk, butter, cream, cheese, whey protein concentrate (WPC), whey protein isolate (WPI), α-lactalbumin (α-La), and β-lactoglobulin (β-Lg)), and may be one of these or a mixture of two or more thereof.

The formula milk according to the present invention may be composed only of the above raw material milk, or may be an aqueous solution, diluted solution, or concentrated solution of the above raw material milk, or may further comprise additional components in addition to the above raw material milk, if necessary. Examples of the additional components include water; foods, food ingredients, or food additives such as soymilk, saccharides such as sugar, sweeteners, fragrances, fruit juices, fruit pulps, vitamins, minerals, oils and/or fats, ceramides, collagen, milk phospholipids, and polyphenols; stabilizers, thickeners, and gelling agents such as pectin, soy polysaccharides, CMC (carboxymethylcellulose), agar, gelatin, carrageenan, and gums, and may be one of these or a mixture of two or more thereof. The formula milk can be prepared by mixing the above components, while optionally with heating and/or optionally with homogenizing. In addition, as the formula milk, heat total sterilized or partial sterilized one can also be used.

### (Fermentation)

In the fermentation step of adding the lactic acid bacterium of the present invention or the lactic acid bacterium composition of the present invention to the aforementioned formula milk, followed by fermentation, conventionally known methods can be appropriately employed without particular limitations. Moreover, the amount of the lactic acid bacteria of the present invention or the lactic acid bacteria composition of the present invention to be added can be appropriately set according to the amount of lactic acid bacteria starter used in the conventionally known method for producing fermented milk, but is, for example, preferably 1 × 10⁷ to 5 × 10⁹ cfu/g, more preferably 1 × 10⁸ to 2 × 10⁹ cfu/g, in a viable bacterial count of the lactic acid bacteria of the present invention, based on the mass of the formula milk.

To the formula milk, in addition to the lactic acid bacterium of the present invention or the lactic acid bacterium composition of the present invention, the additional lactic acid bacteria, yeast, bifidobacteria, and the like may be added. Among these, when the lactic acid bacterium of the present invention is a lactic acid bacterium of the genus Lactobacillus, it is preferable that a lactic acid bacterium of the genus Streptococcus is further added as the other lactic acid bacterium in the method for producing fermented milk of the present invention, and it is more preferable that a lactic acid bacterium belonging to Streptococcus thermophilus is further added. Furthermore, in the case of further adding additional lactic acid bacteria (preferably lactic acid bacteria of the genus Streptococcus), the ratio of the amount added of the lactic acid bacteria of the present invention and the amount added of the additional lactic acid bacteria is preferably 1:0.1 to 1:100, more preferably 1:1 to 1:10, in a bacterial count ratio (in terms of viable bacterial count, hereinafter the same). Furthermore, when the lactic acid bacterium of the present invention is a combination of a lactic acid bacterium of the genus Lactobacillus as the lactic acid bacterium of the present invention and a lactic acid bacterium of the genus Streptococcus as the lactic acid bacterium of the present invention, the ratio of the amount added of the lactic acid bacteria of the genus Lactobacillus to the amount added of the lactic acid bacteria of the genus Streptococcus is preferably 1:0.1 to 1:100, more preferably 1:1 to 1:10, in bacterial count ratio (in terms of viable bacterial count, hereinafter the same).

The fermentation conditions are not particularly limited, and can be appropriately selected depending on the growth conditions of the lactic acid bacteria to be added, the amount of the formula milk, and the like. For example, under aerobic or anaerobic conditions at a temperature of 35 to 45°C and more preferably at a temperature of 38 to 43°C, the mixture is allowed to stand or stirred (preferably stand) for usually 2 to 24 hours, more preferably 3 to 8 hours, and further preferably 4 to 6 hours until the pH of the formula milk added with the lactic acid bacteria of the present invention or the lactic acid bacteria composition of the present invention is 4.7 or less, more preferably 4.6 or less, and further preferably 4.0 to 4.5.

The fermented milk of the present invention can be obtained by the above fermentation. The fermented product after the fermentation step can be used as the fermented milk of the present invention as it is or by concentrating, diluting, drying, freezing, or the like as necessary. Also, the fermented milk of the present invention may be obtained by disrupting or heat-treating the lactic acid bacteria in the fermented product, or by concentrating, diluting, drying, freezing, or the like as necessary. Therefore, the method for producing fermented milk of the present invention may further include these steps (such as concentration step, dilution step, drying step, freezing step, disrupting step, and heat treatment step).

### [Examples]

Hereinafter, the present invention will be described in more detail based on the Examples and Comparative Examples, but the present invention is not limited to the following Examples. Note that in each of the Test Examples below, the expression "%" indicates weight/volume (w/v: g/mL) percent unless otherwise specified.

### (Test Example 1)

(1) The following three lactic acid bacterial strains were used:
   P2103001 strain (Comparative Example 1):
   Lactobacillus delbrueckii subsp. bulgaricus. The bacterial strain is preserved by the Meiji Innovation Center of Meiji Co., Ltd. (postal code 192-0919, 1-29-1 Nanakuni, Hachioji City, Tokyo, Japan);
   Strain f (Example 1): Lactobacillus delbrueckii subsp. bulgaricus identified by accession number NITE BP-03574;
   Strain i (Example 2): Lactobacillus delbrueckii subsp. bulgaricus identified by accession number NITE BP-03575.
(2) Acid tolerance test

Each lactic acid bacterial strain (P2103001 strain, strain f, strain i) was activation-cultured in MRS medium at 37°C under anaerobic conditions for 18 hours. The bacterial solution after activation culture, in an amount of 50 µL, was inoculated into small test tubes each containing 5 mL of MRS medium, and further cultured at 37°C under anaerobic conditions for 18 hours; then, the tubes were centrifuged at 3000 rpm and 4°C for 10 minutes to collect precipitates (bacterial body pellets). The collected precipitates were suspended in 0.85% physiological saline and centrifuged again under the above conditions; after the operation of removing supernatant (washing) was repeated twice, 5 mL of 0.85% physiological saline was added to the precipitates after removal of the supernatant to prepare bacterial body suspensions. Also, the pH of an aqueous solution as a mixture of 0.2% NaCl and 0.175% pepsin (1:10000, FUJIFILM Wako Pure Chemical Corporation) was adjusted with hydrochloric acid (1 N), and filter sterilization was performed to prepare artificial gastric juice of pH 3.0.

To 9 mL of the artificial gastric juice, 1 mL of the bacterial body suspension was added, followed by acid treatment at 37°C for 2 hours. The solution after acid treatment was collected in an amount of 1 mL, neutralized by addition of 9 mL of 67 mM phosphate buffer solution (pH 6.5), and used as acid-treated bacterial solution. The bacterial body suspension and acid-treated bacterial solution were each serially diluted with PBS, then 100 µL each was spread onto agar medium and cultured at 37°C under anaerobic conditions for 48 hours. Viable bacterial counts in each solution were determined from the number of colonies observed after culture and the dilution factor, and the survival rate was calculated by the following formula: Survival rate (%) = (Viable bacterial count in acid- treated bacterial solution × 100/Viable bacterial count in bacterial body suspension) × 100.

As a result, while the survival rate of the P2103001 strain was 2.58%, which is comparable to that of conventional general lactic acid bacteria, the survival rates of strain f and strain i were 65.8% and 95.4%, respectively, indicating that strains f and i have high tolerance to artificial gastric juice (acid).

### (3) Draft genome sequencing

### [Genomic DNA Extraction]

Each lactic acid bacterial strain (P2103001 strain, strain f, strain i) was cultured in MRS medium at 37°C under anaerobic conditions for 18 hours; then, 1 mL of the culture fluid was collected and centrifuged at 13000g and room temperature for 2 minutes to collect precipitates (bacterial body pellets). The collected precipitates were suspended in 10 mM Tris-HCl (pH 8.0) and centrifuged again under the above conditions; after the operation of removing supernatant (washing) was repeated twice, genomic DNA was extracted using the Wizard Genomic DNA Purification Kit (Promega). The DNA concentration was measured using Qubit (manufactured by Thermo Fisher Scientific), and the genomic DNA sample was obtained by diluting to 0.2 ng/µL with ultrapure water.

### [Acquisition of Draft Genomes on Miseq]

First, from the genomic DNA sample obtained above, a DNA library was prepared using the Nextera XT DNA Library Prep kit (manufactured by Illumina) and purified using AMPure XP (manufactured by Beckman Coulter Life Sciences). The average library size was measured using the High Sensitivity DNA kit (manufactured by Agilent), and the concentration was measured using the Quan-iT dsDNA High Sensitivity Assay kit (manufactured by Thermo Fisher Scientific). Then, the solutions were diluted with ultrapure water such that the DNA concentration became 2 nM; all samples were mixed in equal amounts, and 0.2 N sodium hydroxide solution was added to the resulting mixture for denaturation treatment. Next, 10 µL of the denatured mixture (denatured DNA) was mixed with 990 µL of HT1 buffer (manufactured by Illumina), followed by equal amount mixing with 0.2 pM PhiX (manufactured by Illumina). Sequencing analysis was conducted using MiSeq Reagent Kit v3 (manufactured by Illumina, 600 cycles) on the next-generation sequencer MiSeq sequencer (manufactured by Illumina).

### [Data Analysis]

The sequence data obtained above were transferred to CLC Genomics Workbench (manufactured by QIAGEN) to prepare contigs and annotate them. The contigs of the P2103001 strain with the low survival rate in the acid tolerance test (2) were used as the reference sequence, and the contigs of strains f and i with high survival rates were mapped to detect gene mutation sites and extract only the mutation sites accompanied by amino acid mutations from the P2103001 strain. Table 2 shows the mutation contents in strains f and i confirmed from the P2103001 strain. The survival rate of each strain is also shown in Table 2.

**[Table 2]**

| Mutation from P2103001 Strain | | | Strain f | Strain i |
|---|---|---|---|---|
| Base Sequence Mutation | | | 92 | 89 |
| Mutation in Code Region | | | 28 | 29 |
| Nonsynonymous Substitution | | | 7 | 9 |
| | Transposase-related | | 4 | 5 |
| | No Annotation | | 2 | 2 |
| | lysA | | 0 | 1 |
| | kup | | 1 | 1 |
| | | Type of Mutation | Nonsense | Frameshift |
| | | coding DNA | c.1850G > A | c.521delT |
| | | amino acid | p.Trp617* | p.Phe174Serfs*16 |
| Survival Rate (%) | | | 65.8 | 95.4 |

As shown in Table 2, strains f and i with high acid tolerance had 7 and 9 mutation sites accompanied by amino acid mutations from the P2103001 strain, respectively, but among these, the only common mutation in the two strains was the mutation in the kup gene. The nucleotide sequence of the kup gene of the P2103001 strain is shown in SEQ ID NO: 2, and the amino acid sequence encoded by this nucleotide sequence is shown in SEQ ID NO: 1. In the kup gene, for strain f, the 1850th G in the nucleotide sequence of the P2103001 strain was substituted with A, and the codon corresponding to the 617th Trp in the amino acid sequence of the P2103001 strain was substituted with a stop codon (nonsense mutation, Table 4 below). For strain i, the 521st T was deleted in the nucleotide sequence of the P2103001 strain, the amino acids at and after the 174th Phe in the amino acid sequence of the P2103001 strain were substituted due to a frameshift mutation, and furthermore, the codon corresponding to the 190th Leu (the 16th amino acid from Phe174) of the amino acid sequence of the P2103001 strain was substituted with a stop codon (Table 6 below). In Table 2, "Transposase-related" indicates a gene encoding a protein related to transposase, "No Annotation" indicates a gene whose information cannot be specified, and "lysA" indicates a gene encoding a phage-derived lysozyme; none of these genes are considered to affect acid tolerance. Therefore, the kup gene was suggested to be a factor involved in acid tolerance.

### (Test Example 2) Analysis of Revertant Mutant Strains

### [Strain f]

Strain f was cultured in 10% skimmed milk powder medium, prepared by blending 10-mass% skimmed milk powder (10 parts by mass of skimmed milk powder and 90 parts by mass of distilled water) with 0.1% Yeast Extract (Gibco), at 37°C under anaerobic conditions for 18 hours, and after culture, 50 µL of the bacterial solution was inoculated into 5 mL of 10% skimmed milk powder medium and subjected to repeated culture operations (subcultures) under the above conditions to induce mutations. Acid tolerance test was carried out as in (2) of Test Example 1 at the 10th subculture, giving strain (f-1) with a significantly decreased survival rate of 0.74%.

Strain f was also cultured in MRS medium at 37°C under anaerobic conditions for 18 hours, and after culture, 50 µL of the bacterial solution was inoculated into 5 mL of MRS medium and subjected to repeated culture operations (subcultures) under the above conditions 10 times to induce mutations. Strain f after the 10th subculture was designated strain f-2, and acid tolerance test was carried out as in (2) of Test Example 1, showing that the survival rate was 37.7% and acid tolerance was maintained.

For the obtained strains f-1 and f-2, except that the contig of strain f was used as the reference sequence, draft genome sequencing was carried out as in (3) of Test Example 1 to detect gene mutation sites and extract only mutation sites accompanied by amino acid mutations from strain f. Table 3 below shows the mutation contents in strains f-1 and f-2 confirmed from strain f. The survival rate of each strain is also shown in Table 3. Table 4 also shows details of mutations at the 617th site in the amino acid sequence (1849th-1851st sites in the nucleotide sequence) in the P2103001 strain, strain f, strain f-1, and strain f-2.

**[Table 3]**

| Mutation from Strain f | | | Milk Medium, 10 Subcultures (f-1) | MRS, 10 Subcultures (f-2) |
|---|---|---|---|---|
| Base Sequence Mutation | | | 92 | 89 |
| Mutation in Code Region | | | 20 | 21 |
| Nonsynonymous Substitution | | | 9 | 6 |
| | Transposase-related | | 2 | 1 |
| | No Annotation | | 6 | 5 |
| | lysA | | 0 | 0 |
| | kup | | 1 | 0 |
| | | Type of Mutation | Revertant | None |
| | | coding DNA | c.1849T > C | None |
| | | amino acid | *617Gln | None |
| Survival Rate (%) | | | 0.74 | 37.7 |

**[Table 4]**

| Lactic Acid Bacterium | Amino Acid | Base Sequence | | |
|---|---|---|---|---|
| | 617 | 1849 | 1850 | 1851 |
| P2103001 Strain | Trp | T | G | G |
| Strain f | * | T | **A** | G |
| Strain f-1 | Gln | **C** | A | G |
| Strain f-2 | * | T | **A** | G |

| | | | | |
|---|---|---|---|---|
| * : stop codon | | | | |

As shown in Tables 3 to 4, strains f-1 and f-2 had 9 and 6 mutation sites accompanied by amino acid mutations from strain f, respectively (nonsynonymous substitutions), and among these, mutations other than the above "Transposase-related," "No Annotation," and "lysA" were only mutations in the kup gene of strain f-1. In strain f-1 with decreased acid tolerance, the 1849th T in the nucleotide sequence of strain f in the kup gene was substituted with C, and the stop codon of strain f was substituted with Gln. Therefore, compared to the amino acid sequence of the P2103001 strain, strain f-1 had only a mutation in which the 617th Trp was substituted with Gln (1 amino acid substitution). On the other hand, in strain f-2 with maintained acid tolerance, no amino acid mutation accompanied by a mutation from strain f was confirmed in the kup gene. That is, it was shown that in strain f-1, acid sensitivity was restored and acid tolerance decreased due to loss of the nonsense mutation of the kup gene in strain f. Therefore, it has been confirmed that the kup gene is a factor involved in acid tolerance and that inhibition of the function thereof improves acid tolerance.

### [Strain i]

Strain i was cultured in MRS medium at 37°C under anaerobic conditions for 18 hours, and after culture, 50 µL of the bacterial solution was inoculated into 5 mL of MRS medium and subjected to repeated culture operations (subcultures) under the above conditions. In the 10th subculture, the acid tolerance test was carried out as in (2) of Test Example 1 to obtain strain i-1 with a significantly decreased survival rate of 0.074%.

For the obtained strain i-1, except that the contig of strain i was used as the reference sequence, draft genome sequencing was carried out as in (3) of Test Example 1 to detect gene mutation sites and extract only mutation sites accompanied by amino acid mutations from strain i. Table 5 below shows the mutation contents in strain i-1 confirmed from strain i. The survival rate of strain i-1 is also shown in Table 5. Table 6 also shows details of mutations at sites 172-190 in the amino acid sequences of the P2103001 strain, strain i, and strain i-1 and at sites 514-525 in the nucleotide sequence (base sequence) corresponding to sites 172-175 of the amino acid sequences, as well as sequence numbers indicating amino acid sites 172-190 (172-189 in strain i) of each amino acid sequence.

**[Table 5]**

| Mutation from Strain i | | | MRS, 10 Subcultures (i-1) |
|---|---|---|---|
| Base Sequence Mutation | | | 125 |
| Mutation in Code Region | | | 42 |
| Nonsynonymous Substitution | | | 18 |
| | Transposase-related | | 8 |
| | No Annotation | | 8 |
| | lysA | | 1 |
| | kup | | 1 |
| | | Type of Mutation | Revertant |
| | | coding DNA | c.515dupA |
| | | amino acid | p.Ala173fs |
| Survival Rate (%) | | | 0.074 |

**[Table 6]**

| Lactic Acid Bacteria | Amino Acid 172/ Base Sequence 514-6 | Amino Acid 173/ Base Sequence 517-9 | Amino Acid 174/ Base Sequence 520-2 | Amino Acid 175/ Base Sequence 523-5 | Amino Acid 176-190 | SEQ ID NO |
|---|---|---|---|---|---|---|
| P2103001 Strain | Lys/AAG | Ala/GCT | Phe/TTC | Gly/GGT | PIMLVWFAFLGVMGL | 5 |
| Strain i | Lys/AAG | Ala/GCT | **Ser**/T**CG** | **Val/GTC** | QSCLSGSPSWASWA* | 6 |
| Strain i-1 | Lys/AA**A** | Gly/GGC | Phe/TTC | Gly/GGT | PIMLVWFAFLGVMGL | 7 |

As shown in Tables 5 to 6, strain i-1 had 18 mutation sites accompanied by amino acid mutations from strain i, but mutations other than the above "Transposase-related," "No Annotation," and "lysA" were only mutations in the kup gene. In strain i-1 with decreased acid tolerance, A was inserted at the 516th site in the nucleotide sequence of the kup gene of strain i, resulting in reversion to the same amino acid sequence as that at and after the 174th Phe in the amino acid sequence of the P2103001 strain due to the frameshift mutation, in addition to substitution of Ala at the 173rd site with Gly. That is, it was shown that in strain i-1, acid sensitivity was restored and acid tolerance decreased due to loss of the frameshift mutation in the kup gene of strain i. Therefore, this has also confirmed that the kup gene is a factor involved in acid tolerance and that inhibition of the function thereof improves acid tolerance.

### [Industrial Applicability]

As described above, according to the present invention, it becomes possible to provide a lactic acid bacterium that has sufficiently excellent tolerance to acids such as gastric acid and a lactic acid bacterium composition comprising the same, a method for producing a lactic acid bacterium with improved tolerance to acids, a method for improving acid tolerance of a lactic acid bacterium, a method for screening a lactic acid bacterium that has sufficiently excellent tolerance to acids, and a method for producing fermented milk using these lactic acid bacteria and/or lactic acid bacterium compositions.

### [Accession Number]

1.
   (1) Identification label: L. delbrueckii subsp. bulgaricus OLL205405
   (2) Accession number: NITE BP-03574
   (3) Deposit date: December 16, 2021
   (4) Depositary institution: National Institute of Technology and Evaluation NITE Patent Microorganisms Depositary
2.
   (1) Identification label: L. delbrueckii subsp. bulgaricus OLL205406
   (2) Accession number: NITE BP-03575
   (3) Deposit date: December 16, 2021
   (4) Depositary institution: National Institute of Technology and Evaluation NITE Patent Microorganisms Depositary

## Claims

1. A method for improving acid tolerance of a lactic acid bacterium, comprising a step of artificially inhibiting a function of a kup gene of the lactic acid bacterium.

2. The method according to claim 1, wherein the lactic acid bacterium is a lactic acid bacterium of the family Lactobacillaceae.

3. The method according to claim 1, wherein the lactic acid bacterium is a lactic acid bacterium of the genus Lactobacillus.

4. A method for producing a lactic acid bacterium with improved acid tolerance, comprising a step of artificially inhibiting a function of a kup gene of a lactic acid bacterium.

5. The production method according to claim 4, wherein the lactic acid bacterium is a lactic acid bacterium of the family Lactobacillaceae.

6. The production method according to claim 4, wherein the lactic acid bacterium is a lactic acid bacterium of the genus Lactobacillus.

7. A lactic acid bacterium in which a function of a gene is inhibited.

8. The lactic acid bacterium according to claim 7, which is a lactic acid bacterium of the family Lactobacillaceae.

9. The lactic acid bacterium according to claim 7, which is a lactic acid bacterium of the genus Lactobacillus.

10. A lactic acid bacterium composition comprising the lactic acid bacterium according to claim 7.

11. The lactic acid bacterium composition according to claim 10, further comprising a lactic acid bacterium of the genus Streptococcus.

12. The lactic acid bacterium composition according to claim 10, which is a lactic acid bacterium starter.

13. The lactic acid bacterium composition according to claim 10, which is fermented milk.

14. A method for screening a highly acid-tolerant lactic acid bacterium, comprising a selection step of selecting a lactic acid bacterium using inhibition of a function of a kup gene as an indicator.

15. A method for producing fermented milk, comprising a fermentation step of adding any selected from the group consisting of the lactic acid bacterium obtained by the production method according to any one of claims 4 to 6, the lactic acid bacterium according to any one of claims 7 to 9, the lactic acid bacterium composition according to any one of claims 10 to 13, and the lactic acid bacterium selected by the screening method according to claim 14, to formula milk comprising raw material milk and performing fermentation to obtain fermented milk.

16. The method for producing fermented milk according to claim 15, further comprising a step of adding a lactic acid bacterium of the genus Streptococcus to the formula milk.
